(19) Europäisches Patentamt
European Patent Office
Office européen des brevets

(11) Veröffentlichungsnummer: **0 012 424**
**B1**

# (12) EUROPÄISCHE PATENTSCHRIFT

(45) Veröffentlichungstag der Patentschrift: **05.08.81**

(51) Int. Cl.³: **C 07 C 102/02,** C 07 C 103/49

(21) Anmeldenummer: **79105089.1**

(22) Anmeldetag: **11.12.79**

(54) **Verfahren zur Herstellung von Beta-Isobutyrylaminocrotonsäureamid.**

(30) Priorität: **14.12.78 DE 2853887**

(43) Veröffentlichungstag der Anmeldung: **25.06.80 Patentblatt 80/13**

(45) Bekanntmachung des Hinweises auf die Patenterteilung: **05.08.81 Patentblatt 81/31**

(84) Benannte Vertragsstaaten: **BE CH DE FR GB IT NL**

(56) Entgegenhaltungen: **FR-A-2 069 567**

(73) Patentinhaber: **Consortium für elektrochemische Industrie GmbH, Zielstattstrasse 20, D-8000 München 70 (DE)**

(72) Erfinder: **Schilling, Bernd, Dr. Dipl.-Chem., Willibaldstrasse 12 b, D-8000 München 21 (DE)**

**EP 0 012 424 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von β-Isobutyrylaminocrotonsäure-amid.

Es ist bekannt, dass das aus Diketen und Ammoniak leicht zugängliche β-Aminocrotonsäureamid durch Einwirkung von Säureanhydriden oder Säurechloriden als Acylierungsmittel N-acyliert werden kann unter Bildung der entsprechenden β-Acylaminocrotonsäureamide. So wird beispielsweise durch Einwirkung von Isobuttersäureanhydrid durch Erhitzen unter Rückfluss in Chloroform β-Isobutyrylaminocrotonsäureamid in einer Ausbeute von 62% erhalten (vgl. JP-AS 68 03.363, ref. C.A. Bd. 69 (1968), Seite 6263).

Dieses Verfahren ist jedoch in der Praxis unwirtschaftlich, sowohl im Hinblick auf das nur schwer zugängliche Isobuttersäureanhydrid, als auch auf die mässigen Ausbeuten und mit hohem Aufwand verbunden, da mehrere Reinigungsmassnahmen erforderlich sind, um die als Nebenprodukt gebildete Isobuttersäure und unumgesetztes Ausgangsmaterial von dem gewünschten Produkt abzutrennen.

Es wurde nun ein Verfahren zur Herstellung von β-Isobutyrylaminocrotonsäureamid gefunden, das die Umsetzung von β-Aminocrotonsäureamid mit einer Isobutyrylverbindung als Acylierungsmittel in etwa stöchiometrischen Mengen, in Gegenwart eines Lösungsmittels unter milden Bedingungen in guten Ausbeuten ermöglicht, ohne dass hierzu aufwendige Reinigungsmassnahmen für die Abtrennung von Nebenprodukten oder unumgesetztem Ausgangsmaterial erforderlich sind.

Das erfindungsgemässe Verfahren ist dadurch gekennzeichnet, dass als Acylierungsmittel Dimethylketen verwendet wird und die Umsetzung im Bereich von Raumtemperatur bis 70 °C ohne äussere Wärmezufuhr durchgeführt wird.

Das erfindungsgemässe Verfahren kann durch die folgende Gleichung veranschaulicht werden:

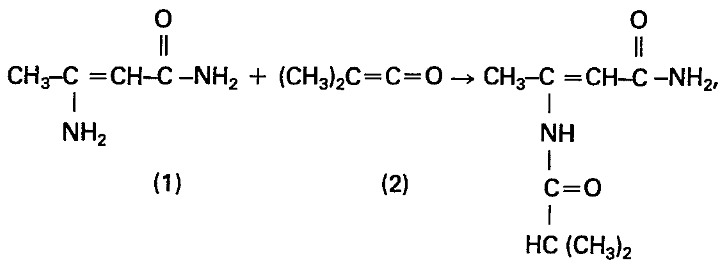

(1) (2)

(3)

wobei die gewünschte Verbindung (3) in Ausbeuten bis zu 90% der Theorie erhalten wird. Daraus ist ersichtlich, dass die Einwirkung von Dimethylketen praktisch ausschliesslich zu einer N-Acylierung führt. Dieses Ergebnis muss insbesondere im Hinblick auf die Untersuchungen von T. Kato und Mitarbeitern in «Heterocycles», Bd. 3, No. 5, 1975, Seite 419 und 421 als überraschend bewertet werden, die beweisen, dass die Einwirkung von Keten auf beta-Aminocrotonsäureamid praktisch ausschliesslich über die Bildung von C-Acylierungsprodukten verläuft.

Bei dem erfindungsgemässen Verfahren fällt die gewünschte Verbindung (3) in Form von 2 Stereoisomeren an, denen vermutlich folgende Konfigurationen zuzuordnen sind:

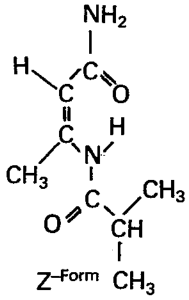

Z-Form

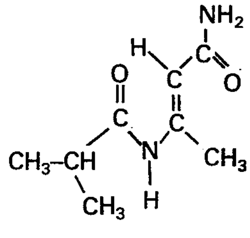

E-Form

Die im folgenden als Z-Form bezeichnete stereoisomere Verbindung (3) hat einen Fp. von 148 °C und ist leicht löslich in Chloroform und Aceton. Sie fällt bei dem erfindungsgemässen Verfahren als Hauptprodukt an. Die als E-Form bezeichnete stereoisomere Verbindung (3) hat einen Fp. von 210 °C und ist schwer löslich in Chloroform und Aceton. Sie fällt bei dem erfindungsgemässen Verfahren in Mengen von 6 bis 18% an, in Abhängigkeit von den gewählten Reaktionsbedingungen, die im folgenden noch näher erläutert werden. Aufgrund ihrer unterschiedlichen Löslichkeit in den genannten Lösungsmitteln können die beiden Stereoisomeren leicht voneinander getrennt werden. Das ist jedoch nicht unbedingt erforderlich, da die E-Form sich beim Erhitzen ab 40 °C in die Z-Form umwandelt.

β-Isobutyrylaminocrotonsäureamid ist ein wertvolles Ausgangsprodukt für die Herstellung von 2-Isopropyl-6-methyl-4-hydroxypyrimidin, das als Zwischenprodukt für die Herstellung von 0,0-Diäthyl-0-(2-isopropyl-6-methyl-pyrimidyl-4)-thionophosphat benötigt wird, das als schnell wirkendes Kontaktinsektizid unter der geschützten Bezeichnung «Diazinon» im Handel ist.

Bei der Herstellung des genannten Pyrimidinderivates wird das β-Isobutyrylaminocrotonsäureamid unter alkalischen Bedingungen erhitzt, wobei durch Ringschluss aus der stereoisomeren Z-Form der Pyrimidinring gebildet wird. Da hierbei auf jeden Fall Temperaturen oberhalb von 40 °C notwendig sind, können für dieses Verfahren die beiden stereoisomeren Formen des β-Isobutyrylaminocrotonsäureamids in gleicher Weise eingesetzt werden, das heisst eine Auftrennung des nach dem erfindungsgemässen Verfahren erhältlichen Gemisches aus den beiden Stereoisomeren ist nicht erforderlich.

Bei Durchführung des erfindungsgemässen Verfahrens wird das β-Aminocrotonsäureamid zweckmässig in einem organischen Lösungsmittel mit einem Siedepunkt oberhalb von 40 °C dispergiert. Beispiele für derartige Dispersionsmedien sind halogenierte aliphatische Kohlenwasserstoffe, wie Chloroform und Tetrachloräthylen, aromatische Kohlenwasserstoffe, wie Toluol und Xylol, substituierte aromatische Kohlenwasserstoffe, wie Chlorbenzol und Nitrobenzol, Carbonsäureester, wie Essigsäureäthylester und Essigsäurebutylester, Carbonsäureanhydride, wie Isobuttersäureanhydrid, Nitrile, wie Acetonitril und Isobutyronitril, Äther, wie Tetrahydrofuran, Dioxan und Diäthylenglykoläther, ferner Ketone, wie Aceton, Methyläthylketon und Cyclohexanon.

Es ist nicht unbedingt erforderlich, dass das verwendete Dispersionsmedium absolut wasserfrei ist. Geringe Wassermengen im Lösungsmittel, worunter bis zu etwa 1 Mol Wasser je Mol eingesetztes β-Aminocrotonsäureamid zu verstehen sind, können toleriert werden. Grössere Wassermengen sind indessen zu vermeiden, da sie eine Ausbeuteverschlechterung zur Folge haben. Es wurde jedoch nachgewiesen, dass die Anwesenheit von Wasser in Mengen bis zu etwa 0,1 Mol je Mol eingesetztes β-Aminocrotonsäureamid zu einer Ausbeutesteigerung führt.

Ausserdem ist es vorteilhaft das Dispersionsmedium durch Zugabe von organischen Säuren abzupuffern um zu vermeiden, dass das stark basische β-Aminocrotonsäureamid die Selbstreaktion des Dimethylketens katalysiert. Als organische Säuren können Carbonsäuren mit vorzugsweise 1 bis 4 C-Atomen, wie Ameisen-, Essig-, Propion- oder Buttersäuren verwendet werden, wobei sich Isobuttersäure besonders bewährt hat. Die zugesetzten Mengen an organischen Carbonsäuren können im Bereich von 0,2 bis 2 Mol Säure, je Mol eingesetztes β-Aminocrotonsäureamid variiert werden.

Der Zusatz einer organischen Säure ist insbesondere bei Verwendung von unpolaren Lösungsmitteln, wie Toluol oder Xylol von Vorteil, in welchen sich das β-Aminocrotonsäureamid nur unvollständig löst, da hierdurch die Löslichkeit des Ausgangsmaterials verbessert und die Ausbeuten an dem gewünschten Produkt erhöht werden. Durch den Säurezusatz wird ferner die Bildung der stereoisomeren E-Form begünstigt.

Bei dem erfindungsgemässen Verfahren werden die Reaktionsteilnehmer, das sind β-Aminocrotonsäureamid und Dimethylketen in etwa stöchiometrischen Mengen eingesetzt. Ein geringer Überschuss an Dimethylketen im Bereich von etwa 0,2 bis 0,4 Mol ist indessen zur Erzielung hoher Ausbeuten an dem gewünschten Endprodukt vorteilhaft. Ein grösserer Überschuss ist jedoch zu vermeiden, da hierbei bereits die Gefahr besteht, dass das gewünschte Endprodukt durch polymere Produkte aus dem zur Selbstreaktion neigenden Dimethylketen verunreinigt wird. Durch einen Unterschuss an Dimethylketen kann zwar eine derartige Selbstreaktion weitgehend unterdrückt werden, das führt aber insbesondere bei einer diskontinuierlichen Verfahrensweise zu einer Verminderung der Ausbeute.

Bei Durchführung des erfindungsgemässen Verfahrens wird das Dimethylketen in gasförmigem oder flüssigem Zustand in das im Lösungsmittel dispergierte β-Aminocrotonsäureamid eingetragen. Die bereits bei Raumtemperatur beginnende Reaktion verläuft exotherm, sodass eine äussere Wärmezufuhr nicht erforderlich ist. Temperaturen bis zu etwa 70 °C sollten jedoch nicht überschritten werden, was durch Kontrolle der Zugabegeschwindigkeit des Dimethylketens und/oder durch Kühlung von aussen erreicht werden kann. Die besten Ergebnisse werden bei Arbeitstemperaturen im Bereich von 40 bis 60 °C erzielt.

Dimethylketen in flüssigem Zustand (Sdp. 34 °C) kann unverdünnt oder gelöst in einem inerten organischen Lösungsmittel eingesetzt werden. Bei unverdünnter Zugabe wird zweckmässig unter Sauerstoffausschluss, das heisst in Gegenwart einer Schutzgasatmosphäre, wie Stickstoff gearbeitet. Beispiele für inerte organische Lösungsmittel sind carbonylgruppenfreie Lösungsmittel, die auch für die Dispergierung des β-Aminocrotonsäureamids verwendet werden können, ferner niedrigsiedende Äther, wie Diäthyläther. Die Reaktion wird üblicherweise unter Normaldruck durchgeführt, wobei durch mechanische Bewegung für eine gute Durchmischung der Reaktionspartner gesorgt werden kann.

Bei Einsatz von Dimethylketen in gasförmigem Zustand wird dieses vorteilhaft unverdünnt unter vermindertem Druck und in Gegenwart einer Schutzgasatmosphäre in das Reaktionsmedium eingetragen, wobei sich ein Unterdruck im Bereich von 50 bis 300 mbar besonders bewährt hat. Die gasförmige Einleitung kann jedoch auch bei Normaldruck oder leichtem Überdruck, wie er durch die Gegenwart einer strömenden Schutzgasatmosphäre erzeugt wird, vorgenommen werden, durch die gasförmige Einleitung erfolgt bereits eine gute Durchmischung der Reaktionspartner, diese kann jedoch gegebenenfalls durch mechanische Bewegung unterstützt werden.

Unter Einhaltung dieser Bedingungen ist die Umsetzung bei diskontinuierlicher Arbeitsweise im allgemeinen nach etwa 15 Minuten bis etwa 4 Stunden beendet, was visuell durch das Verschwinden der gelben Farbe des Dimethylketens beobachtet werden kann. Das Verfahren kann jedoch auch kontinuierlich durchgeführt werden, wobei Kontaktzeiten von etwa 15 Minuten nicht überschritten werden sollten.

Nach beendeter Umsetzung wird das gebildete

Isomerengemisch abfiltriert bzw. unter vermindertem Druck von flüchtigen Bestandteilen befreit und gegebenenfalls durch Umkristallisieren gereinigt.

In den folgenden Beispielen wird das erfindungsgemässe Verfahren näher erläutert.

Beispiel 1

Ein mit Rückflusskühler, Tropftrichter und Rührvorrichtung ausgerüstetes Reaktionsgefäss wurde mit einer Lösung von 10 g (0,1 Mol) β-Aminocrotonsäureamid in 100 ml Diäthylenglykoldimethyläther beschickt. Dann wurden unter Rühren innerhalb von 10 Minuten 40 ml einer Lösung von Dimethylketen (0,12 Mol) in Diäthyläther tropfenweise eingetragen, wobei die Temperatur auf etwa 50 °C anstieg. Nach beendeter Zugabe wurde das ausgefallene E-β-Isobutyrylaminocrotonsäureamid abfiltriert (1,1 g = 6,5% Ausbeute) und die zurückbleibende Lösung unter vermindertem Druck von flüchtigen Bestandteilen befreit. Der Rückstand wurde mit Wasser gewaschen und mehrmals mit Chloroform extrahiert. Dann wurde das Lösungsmittel aus der organischen Phase abgezogen und der Rückstand aus Toluol umkristallisiert. Es wurden 13,4 g (= 78,8% Ausbeute) farbloses, kristallisiertes Z-β-Isobutyrylaminocrotonsäureamid erhalten.

Die Gesamtausbeute an β-Isobutyrylaminocrotonsäureamid betrug 85,3% der Theorie, bezogen auf eingesetztes β-Aminocrotonsäureamid.

Analytische Daten:

E-β-Isobutyrylaminocrotonsäureamid:

Fp. 210 °C

NMR-Spektrum (DMSO, -$d_6$-TMS als int. Stand)

1,09, d, J=6,5 Hz, 6 H; 2,28, S, 3H; 2,6; Sept., J=6,5 Hz, 1H; 6,72, S, 1H; 6,81 d, J= 50 Hz, 2H; 9,18, S, 1H;

Z-β-Isobutyrylaminocrotonsäureamid:

Fp. 148 °C

NMR-Spektrum (DMSO, -$d_6$-TMS als int. Stand)

1,14, d, J=6,5 Hz, 6H; 2,26, S, 3H; 2,38 Sept., J=6,5 Hz, 1H; 4,94, S, 1H; 7,10 d=42 Hz, 2H; 12,31, S, 1H.

Beispiel 2

Das in Beispiel 1 beschriebene Verfahren wurde wiederholt mit der Abänderung, dass das β-Aminocrotonsäureamid in 100 ml Aceton gelöst wurde.

Es wurden 1,7 g, = 10% Ausbeute E-β-Isobutyrylaminocrotonsäureamid und 13,7 g=80,6% Ausbeute Z-β-Isobutyrylaminocrotonsäureamid erhalten.

Die Gesamtausbeute an β-Isobutyrylaminocrotonsäureamid betrug 90,6% der Theorie.

Beispiel 3

Das in Beispiel 1 beschriebene Verfahren wurde wiederholt mit folgenden Abänderungen:

6 g (0,06 Mol) β-Aminocrotonsäureamid wurden in 60 ml Nitrobenzol gelöst. Dann wurden unter Rühren innerhalb von 10 Minuten 5,88 g (0,084 Mol) Dimethylketen, verdünnt mit 10 ml Nitrobenzol tropfenweise eingetragen. Nach beendeter Zugabe wurde das Reaktionsgemisch 24 Stunden bei Raumtemperatur stehen gelassen. Dann wurde der ausgefallene kristalline Feststoff abfiltriert und mit niedrigsiedendem Petroläther gewaschen. Es wurden 8 g (=78,4% Ausbeute) Z/E-Isomerengemisch erhalten. Aus der Mutterlauge wurden durch Ausfällen mit Petroläther und Umkristallisieren aus Toluol weitere 1,15 g (=11,3% Ausbeute) Z-Isomer erhalten.

Die Gesamtausbeute an β-Isobutyrylaminocrotonsäureamid betrug 89,7% der Theorie.

Beispiel 4

Das in Beispiel 1 beschriebene Verfahren wurde wiederholt mit folgenden Abänderungen:

10 g (0,1 Mol) β-Aminocrotonsäureamid wurden in 100 ml Chloroform gelöst und mit 1,85 ml (0,02 Mol) Isobuttersäure versetzt. Dann wurden unter Rühren 40 ml einer Lösung von Dimethylketen (0,14 Mol) in Diäthyläther innerhalb von 10 Minuten tropfenweise eingetragen.

Es wurden 3,2 g (=18,8%) E-β-Aminocrotonsäureamid, 11,3 g (=66,5%) Z-β-Isobutyrylaminocrotonsäureamid erhalten.

Die Gesamtausbeute betrug 85,3% der Theorie.

Beispiel 5

Das in Beispiel 1 beschriebene Verfahren wurde wiederholt mit folgenden Abänderungen:

10 g (0,1 Mol) β-Aminocrotonsäureamid wurden in 100 ml Toluol suspendiert. Dann wurden unter Rühren innerhalb von 10 Minuten 9,1 g (0,13 Mol) Dimethylketen, verdünnt mit 10 ml Toluol tropfenweise eingetragen. Nach beendeter Zugabe wurde das ausgefallene E/Z-Isomerengemisch abfiltriert und mit Petroläther nachgewaschen. Die Gesamtausbeute betrug 14,2 g=83,5% der Theorie.

Beispiel 6

Ein mit Rückflusskühler, Tropfentrichter und Gaseinleitungsrohr ausgerüstetes Reaktionsgefäss wurde mit einer Lösung von 4 g (0,04 Mol) β-Aminocrotonsäureamid in 40 ml Isobuttersäureanhydrid und 3,7 ml (0,04 Mol) Isobuttersäure beschickt. Dann wurde das Reaktionsgefäss mit Stickstoff gespült und innerhalb von 5 Minuten 5 ml (0,046 Mol) Dimethylketen unter Stickstoff tropfenweise eingetragen, wobei die Temperatur bis auf etwa 70 °C anstieg. Nach beendeter Zugabe und Abkühlen des Reaktionsgemisches wurde das ausgefallene Z/E-Isomerengemisch abfiltriert und mit Petroläther nachgewaschen.

Ausbeute: 2,8 g=41,2%.

Aus der Mutterlauge wurden durch Ausfällen mit Petroläther weitere 3,2 g=47,1% Z-Isomer gewonnen.

Die Gesamtausbeute betrug 88,3% der Theorie.

Beispiel 7

Das in Beispiel 6 beschriebene Verfahren wurde wiederholt mit der Abänderung:

4 g (0,04 Mol) β-Aminocrotonsäureamid wurden in 40 ml Isobutyronitril und 3,7 ml (0,04 Mol) Isobuttersäure gelöst.

Es wurden 2,4 g=35,3% Z/E-Isomerengemisch

abfiltriert und aus der Mutterlauge wurden 3,6 g=52,9% Z-Isomer gewonnen.

Die Gesamtausbeute an β-Isobutyrylaminocrotonsäureamid betrug 88,2% der Theorie.

Beispiel 8

Ein mit Gaseinleitung ausgerüstetes evakuierbares Reaktionsgefäss wurde unter Stickstoff mit einer Lösung von 30 g (0,3 Mol) β-Aminocrotonsäureamid in 300 ml Diäthylenglykoldimethyläther beschickt. Dann wurden unter einem Unterdruck von 100 mbar innerhalb von 4 Stunden 25 g (0,35 Mol) Dimethylketen gasförmig eingeleitet, wobei die Temperatur auf etwa 35 °C anstieg. Anschliessend wurde das Reaktionsgefäss belüftet und aus dem Reaktionsgemisch 9 g=17,6% E-β-Isobutyrylaminocrotonsäureamid abfiltriert. Aus der Lösung wurden nach Abdestillieren des Lösungsmittels und Umkristallisieren des Rückstandes aus Toluol 34,5 g=67,6% Z-Isomer erhalten.

Die Gesamtausbeute an β-Isobutyrylaminocrotonsäureamid betrug 85,2% der Theorie.

Beispiel 9

Das in Beispiel 8 beschriebene Verfahren wurde wiederholt mit der Abänderung, dass β-Aminocrotonsäureamid in 300 ml Isobuttersäureanhydrid dispergiert wurde.

Aus der Lösung wurden 31,2 g=61,2% Z/E-Isomer abfiltriert. Durch Aufarbeiten der Mutterlauge wurden 13,3 g=26,1% Z-Isomer isoliert.

Die Gesamtausbeute an β-Isobutyrylaminocrotonsäureamid betrug 87,3% der Theorie.

Beispiel 10

Ein mit Gasdurchleitung ausgerüstetes Druckgefäss wurde mit einer Suspension von 10 g (0,1 Mol) β-Aminocrotonsäureamid in 100 ml Xylol beschickt. Dann wurden innerhalb von 2 Stunden 9 g (0,128 Mol) gasförmiges Diketen in einer strömenden Stickstoffatmosphäre durchgeleitet. Anschliessend wurde das Reaktionsgefäss belüftet und aus der Lösung 13,8 g Z/E-Isomerengemisch abfiltriert.

Die Gesamtausbeute an β-Isobutyrylaminocrotonsäureamid betrug 81,2% der Theorie.

Beispiel 11

Nach dem in Beispiel 1 beschriebenen Verfahren wurden jeweils 4 g (0,04 Mol) β-Aminocrotonsäureamid, gelöst in 40 ml Diäthylenglykoldimethyläther und 3,52 g (0,04 Mol) Isobuttersäure mit 5 ml (0,046 Mol) Dimethylketen in Gegenwart unterschiedlicher Wassermengen umgesetzt. Nach der Aufarbeitung gemäss Beispiel 1 wurden folgende Ausbeuten erzielt:

a) ohne Wasserzusatz
4,6 g=67,5% Z-Isomer; 0,9 g=13,2% E-Isomer;
Gesamtausbeute: 80,7%
b) mit 0,072 g $H_2O$ (0,004 Mol)
4,7 g=69,1% Z-Isomer; 1,1 g=16,2% E-Isomer;
Gesamtausbeute: 85,3%
c) mit 0,36 g $H_2O$ (0,02 Mol)
4,4 g=64,7% Z-Isomer; 1,0 g=14,7% E-Isomer;
Gesamtausbeute: 79,4%
d) mit 0,72 g $H_2O$ (0,04 Mol)
4,3 g=63,2% Z-Isomer; 0,9 g=13,2% E-Isomer;
Gesamtausbeute: 76,4%
e) mit 1,44 g $H_2O$ (0,08 Mol)
3,8 g=55,8% Z-Isomer; 0,3 g=4,4% E-Isomer;
Gesamtausbeute: 60,2%.

**Patentansprüche**

1. Verfahren zur Herstellung von β-Isobutyrylaminocrotonsäureamid durch Umsetzung von β-Aminocrotonsäureamid mit einer Isobutyrylverbindung als Acylierungsmittel in etwa stöchiometrischen Mengen, in Gegenwart eines Lösungsmittels unter Erhitzen, dadurch gekennzeichnet, dass als Acylierungsmittel Dimethylketen verwendet und die Umsetzung im Bereich von Raumtemperatur bis 70 °C ohne äussere Wärmezufuhr durchgeführt wird.

2. Verfahren nach Anspruch 1, dadurch gekennzeichnet, dass das Dimethylketen in das in einem organischen Lösungsmittel mit einem Siedepunkt oberhalb von 40 °C dispergierte β-Aminocrotonsäureamid eingetragen wird.

3. Verfahren nach Anspruch 2, dadurch gekennzeichnet, dass das verwendete Dispersionsmedium 0,1 Mol Wasser je Mol eingesetztes β-Aminocrotonsäureamid enthält.

4. Verfahren nach Anspruch 2, dadurch gekennzeichnet, dass das verwendete Dispersionsmedium 0,2 bis 2 Mol einer Carbonsäure mit 1 bis 4 C-Atomen enthält.

5. Verfahren nach Anspruch 1 bis 4, dadurch gekennzeichnet, dass Dimethylketen in einem molaren Überschuss von 0,2 bis 0,4 Mol eingesetzt wird.

6. Verfahren nach Anspruch 1 bis 5, dadurch gekennzeichnet, dass Dimethylketen in flüssigem Zustand unverdünnt unter Sauerstoffausschluss in Gegenwart einer Schutzgasatmosphäre bei Normaldruck eingetragen wird.

7. Verfahren nach Anspruch 1 bis 5, dadurch gekennzeichnet, dass Dimethylketen in gasförmigem Zustand unverdünnt unter vermindertem Druck im Bereich von 50 bis 300 mbar in Gegenwart einer Schutzgasatmosphäre eingetragen wird.

8. Verfahren nach Anspruch 1 bis 5, dadurch gekennzeichnet, dass Dimethylketen in gasförmigem Zustand in Gegenwart einer strömenden Schutzgasatmosphäre eingetragen wird.

**Claims**

1. Process for the manufacture of β-isobutyrylaminocrotonic acid amide by reacting β-aminocrotonic acid amide with an isobutyryl compound as acylating agent in approximately stoichiometric amounts, in the presence of a solvent and with heating, characterized in that dimethylketene is used as the acylating agent and the reaction is carried out in the range of from room temperature to 70 °C without external application of heat.

2. Process according to claim 1, characterized in that the dimethylketene is introduced into the β-aminocrotonic acid amide dispersed in an

organic solvent that has a boiling point above 40 °C.

3. Process according to claim 2, characterized in that the dispersion medium used contains 0.1 mole of water per mole of β-aminocrotonic acid amide.

4. Process according to claim 2, characterized in that the dispersion medium used contains 0.2 to 2 moles of a carboxylic acid having 1 to 4 carbon atoms.

5. Process according to claims 1 to 4, characterized in that the dimethylketene is used in a molar excess of from 0.2 to 0.4 mole.

6. Process according to claims 1 to 5, characterized in that liquid dimethylketene is introduced, undiluted, in the absence of oxygen and in the presence of a protective gas atmosphere at normal pressure.

7. Process according to claims 1 to 5, characterized in that gaseous dimethylketene is introduced, undiluted, under a reduced pressure in the range of 50 to 300 mbar, in the presence of a protective gas atmosphere.

8. Process according to claims 1 to 5, characterized in that gaseous dimethylketene is introduced in the presence of a flowing protective gas atmosphere.

**Revendications**

1. Procédé de préparation du β-isobutyryl-amino-crotonamide par réaction du β-amino-crotonamide avec un composé isobutyrylique comme agent d'acylation en des quantités à peu près stoechiométriques, en présence d'un solvant, avec chauffage, procédé caractérisé en ce qu'on utilise le diméthylcétène comme agent d'acylation et on effectue la réaction dans un intervalle de température allant de la température ambiante à 70 °C, sans apport de chaleur extérieur.

2. Procédé selon la revendication 1, caractérisé en ce qu'on introduit le diméthylcétène dans le β-amino-crotonamide dispersé au sein d'un solvant organique ayant un point d'ébullition supérieur à 40 °C.

3. Procédé selon la revendication 2, caractérisé en ce que le milieu de dispersion utilisé renferme 0,1 mole d'eau par mole du β-amino-crotonamide mis en jeu.

4. Procédé selon la revendication 2, caractérisé en ce que le milieu de dispersion utilisé contient de 0,2 à 2 moles d'un aide carboxylique renfermant de 1 à 4 atomes de carbone.

5. Procédé selon l'une quelconque des revendications 1 à 4, caractérisé en ce que le diméthylcétène est mis en jeu en un excès molaire de 0,2 à 0,4 mole.

6. Procédé selon l'une quelconque des revendications 1 à 5, caractérisé en ce qu'on introduit le diméthylcétène à l'état liquide, non dilué, à l'abri de l'oxygène, en présence d'une atmosphère gazeuse protectrice, sous la pression normale.

7. Procédé selon l'une quelconque des revendications 1 à 5, caractérisé en ce qu'on introduit le diméthylcétène à l'état gazeux, non dilué, sous pression réduite, dans un intervalle de pression allant de 5 à 30 kPa, en présence d'une atmosphère gazeuse protectrice.

8. Procédé selon l'une quelconque des revendications 1 à 5, caractérisé en ce qu'on introduit le diméthylcétène, à l'état gazeux, en présence d'une atmosphère gazeuse protectrice en écoulement.